# EUROPÄISCHE PATENTSCHRIFT

(11) **EP 3 112 861 B1**
(45) Veröffentlichungstag und Bekanntmachung des Hinweises auf die Patenterteilung: **29.08.2018**
(21) Anmeldenummer: 15175186.4
(22) Anmeldetag: 03.07.2015
(51) Int. Cl.: G01N 33/487, G01N 35/10

(54) **VERFAHREN ZUR BERÜHRUNGSERKENNUNG EINER PIPETTIERNADEL**
METHOD FOR TOUCH DETECTION FOR A PIPETTING NEEDLE
PROCÉDÉ DE DÉTECTION DE CONTACT D'UNE AIGUILLE DE PIPETAGE

(43) Veröffentlichungstag der Anmeldung: 04.01.2017
(73) Patentinhaber: Siemens Healthcare Diagnostics Products GmbH, 35041 Marburg (DE)
(72) Erfinder: Wiedekind-Klein, Alexander, 56812 Cochem (DE)

(56) Entgegenhaltungen:
- EP-A1- 0 913 671
- AU-A1- 2005 211 570
- DE-A1-102006 052 833
- US-A- 4 326 851
- US-A- 5 365 783
- US-A- 5 648 727

## Beschreibung

Die Erfindung betrifft ein Verfahren zur Berührungserkennung einer Pipettiernadel in einem In-Vitro-Diagnostiksystem.

Zahlreiche Nachweis- und Analyseverfahren zur Bestimmung physiologischer Parameter in Körperflüssigkeitsproben oder biologischer Proben werden heute automatisiert in großer Anzahl in entsprechenden In-Vitro-Diagnostiksystemen durchgeführt. Hierfür werden für Proben, Reagenzien und auch für die eigentliche Nachweisreaktion geeignete, auch als Küvetten bezeichnete Gefäße verwendet. Die Blutproben werden dem Gerät in Blutentnahmeröhrchen zugeführt. Blutentnahmeröhrchen werden üblicherweise aus transparentem Kunststoff oder Glas gefertigt und sind an der Spitze mit einem speziellen Anschluss für Kanülen ausgestattet.

Heutige In-Vitro-Diagnostiksysteme sind in der Lage, eine Vielzahl von Nachweisreaktionen und Analysen mit einer Probe durchzuführen. Hierzu umfassen derartige Geräte üblicherweise eine Aufnahmeposition für ein Reaktionsgefäß sowie ein der Aufnahmeposition zugeordnetes Analysesystem. Um eine Vielzahl von Untersuchungen automatisiert durchführen zu können, ist es an einer Vielzahl von Stellen erforderlich, durch automatisiertes Pipettieren kleine Flüssigkeitsmengen aus den entsprechenden Behältnissen zu entnehmen. So müssen beispielsweise Aliquots der Blutprobe aus den Blutentnahmeröhrchen oder genau vorgegebene Teilmengen von Reagenzien aus den Reagenzbehältern entnommen und in das für die jeweilige Untersuchung bestimmte Reaktionsgefäß transferiert werden. Hierfür sind je nach Einsatzzweck mehrere entsprechende Pipettiersysteme in dem System vorgesehen.

Ein derartiges Pipettiersystem weist üblicherweise an einem aktiv beweglichen Element wie z. B. einem Transportarm oder Schwenkarm eine Pipettiernadel auf, die in einer Nadelhalterung am Pipettierarm befestigt ist. Die Pipettiernadel ist als Hohlnadel ausgestaltet, die durch Druck oder Unterdruck betrieben mit und ohne Steuerflüssigkeiten automatisiert definierte Probenmengen entnehmen kann. Die Pipettiernadel wird entlang der Mittelachse des jeweiligen Gefäßes eingeführt, durchsticht bei verschlossenen Gefäßen gegebenenfalls einen elastischen Verschlussstopfen und wird in die Flüssigkeit eingetaucht. Das Eintauchen, d. h. die Berührung der Flüssigkeitsoberfläche wird mittels eines entsprechenden Berührungserkennungseinrichtung erkannt und die vorgegebene Menge druckgesteuert eingesaugt. Die entnommene Menge wird dann der entsprechenden Analyse zugeführt. Anschließend wird die Pipettiernadel in einer entsprechenden Einrichtung gespült und ist für den nächsten Einsatz bereit.

Beim oben beschriebenen Vorgang ist die zuverlässige Erkennung der Berührung der Oberfläche der Flüssigkeit unbedingt notwendig, um zum einen den Füllstand der Flüssigkeit festzustellen und zum anderen sicher zu stellen, dass keine Luft pipettiert wird. Problematisch ist hierbei, dass die zu detektierenden Flüssigkeitsmengen extrem klein sind (z. T. wenige µl), so dass bekannte Techniken der Füllstandsmessung wie z. B. über Schwimmer, Induktivität oder Leitfähigkeit kaum einsetzbar sind.

Bislang wurde daher in In-Vitro-Diagnostiksystemen in der Regel eine kapazitive Messung verwendet, welche als reine analoge Schaltungstechnik auf Basis von Operationsverstärkern realisiert war. Die notwenige Empfindlichkeit konnte nur durch Detektion von Kapazitätsänderungen erreicht werden, die dann durch einen Spannungsimpuls an die Steuerelektronik gemeldet wurden.

Nachteilig ist dabei, dass auftretende Störungen, wie zum Beispiel elektrostatische Entladungen und Hochfrequenzfelder ebenfalls solche Spanungsimpulse erzeugen können. Es gibt daher bislang keine Möglichkeit, in bekannten Systemen Störung und echten Spannungsimpuls als Berührungssignal zu unterscheiden.

DE 10 2006 052 833 beschreibt ein Verfahren zum Feststellen einer Verstopfung der Aufnahmeöffnung einer Pipettiernadel. Es ist daher Aufgabe der Erfindung, ein Verfahren der eingangs genannten Art anzugeben, welches eine zuverlässigere Berührungserkennung der Pipettiernadel erlaubt und besonders störungsunempfindlich ist.
Diese Aufgabe wird erfindungsgemäß gelöst, indem
- die Pipettiernadel zyklisch durch eine zwischen Pipettiernadel und einem Referenzpotenzial angelegte elektrische Spannung oder Strom aufgeladen und durch anschließende elektrische Verbindung der Pipettiernadel mit dem Referenzpotenzial wieder entladen wird,
- aus einer Anzahl von während des Aufladens und/oder Entladens erfasster Messgrößen eine für die aktuelle Kapazität zwischen Pipettiernadel und Referenzpotenzial charakteristische Größe ermittelt wird, und
- der zeitliche Verlauf der Größe anhand einer Anzahl vorgegebener Kriterien kontinuierlich überprüft wird und bei Erfüllung der vorgegebenen Kriterien ein Berührungssignal erzeugt wird, dadurch gekennzeichnet, dass als die für die Kapazität zwischen Pipettiernadel (18) und Referenzpotenzial charakteristische Größe die Anzahl der Auf- und Entladevorgänge innerhalb eines vorbestimmten Zeitraums verwendet wird.
Die Erfindung geht dabei von der Überlegung aus, dass eine zuverlässigere Berührungserkennung dadurch erreichbar wäre, dass nicht nur eine punktuelle relative, sondern eine kontinuierliche absolute kapazitive Messung durchgeführt wird. Dies ist besonders einfach erreichbar, indem die Kapazität zwischen Pipettiernadel und deren Umgebung, die ein Referenzpotenzial (z. B. dem Erd- oder Gehäusepotenzial) darstellt, als Referenzgröße herangezogen wird. Durch Betrachtung von Messwerten bei zyklischen Auflade- und Entladevorgängen der Pipettiernadel kann eine geeignete, für die aktuelle Kapazität zwischen Pipettiernadel und Referenzpotenzial charakteristische Größe ermittelt werden, da die beim Aufladevorgang mit vorgegebener Spannung oder Strom übertragene Ladung von der Kapazität abhängig ist. Diese Größe, die die Kapazität zwischen der Pipettiernadel und deren Umgebung repräsentiert, kann als Basisgröße für kontinuierliche Überprüfungen herangezogen werden und es können geeignete Kriterien zur Erkennung einer Berührung vorgegeben werden. Für die Anwendung im Bereich der Füllstandsdetektion ist eine Kalibrierung der Schaltung auf eine absolute Größe in Farad nämlich nicht notwendig. Wie hier beschrieben kann es sich jedoch beispielsweise bei der für die Kapazität charakteristischen Größe um die Kapazität selbst handeln, die beispielsweise in der SI-Einheit Farad angegeben ist.
Eines der vorgegebenen Kriterien ist dabei vorteilhafterweise dadurch gegeben, dass innerhalb eines vorgegebenen Zeitraumes eine Erhöhung der Größe um einen Mindestwert erfolgt. Die gemessene Kapazität wird nämlich beim Eintauchen der Pipettiernadel größer. Dieser Anstieg der die Kapazität charakterisierenden Größe kann durch Vorgabe einer Steigerung in einem bestimmten Zeitraum erkannt werden.
Dieser bestimmte, vorgegebene Zeitraum beträgt vorteilhafterweise weniger als 10 ms, insbesondere weniger als 5 ms. Die Änderung der Kapazität erfolgt nämlich beim Eintauchen in die Flüssigkeit sprunghaft, so dass innerhalb dieses kurzen Zeitraums eine deutliche Steigerung der Kapazität messbar ist.
Bezüglich der Höhe dieses Sprunges der Kapazität kann im Verfahren vorteilhafterweise ein fester Mindestwert vorgegeben werden. Dieser kann z. B. vorab anhand von Versuchsmessungen bestimmt werden.

In alternativer vorteilhafter Ausgestaltung des Verfahrens kann der Mindestwert aber auch dynamisch in Abhängigkeit von der Stärke eines Rauschens der für die Kapazität charakteristischen Größe bestimmt werden. Er kann beispielsweise einem Faktor der Stärke des Grundrauschens der Größe oder der zu erwartenden Flüssigkeitsmenge entsprechen.

Weiterhin ist vorteilhafterweise ein zusätzliches Kriterium zur Erkennung der Berührung der Flüssigkeitsoberfläche dadurch gegeben, dass die Größe im zeitlichen Anschluss an den vorgegebenen Zeitraum des Sprunges für einen zweiten vorgegebenen Zeitraum innerhalb eines Bereichs konstant bleibt. Der Kapazitätsverlauf ist nämlich neben dem Sprung beim Berühren der Oberfläche dadurch gekennzeichnet, dass er nach dem Sprung auf dem neuen Wert verharrt. Hierfür kann ein Wertefenster mit einer oberen und einer unteren Abweichung vorgegeben werden, innerhalb dessen der Wert für einen bestimmten Zeitraum verbleiben muss.

Dieser zweite vorgegebene Zeitraum des Verharrens beträgt vorteilhafterweise mehr als 10 ms. Hierdurch kann insbesondere ausgeschlossen werden, dass die Berührungserkennung fehlerhaft durch einen z. B. durch elektromagnetische Störsignale verursachten Sprung anspricht, da solche Signale in der Regel kürzer andauern.

In einer ersten vorteilhaften Ausgestaltung kann für das genannte Wertefenster analog zur Sprunghöhe ein fester Bereich vorgegeben werden. Dieser kann wiederum vorab mit Testmessungen bestimmt werden.

Alternativ kann der Bereich vorteilhafterweise ebenso dynamisch in Abhängigkeit von der Stärke eines Rauschens der für die Kapazität charakteristischen Größe oder der zu erwartenden Flüssigkeitsmenge bestimmt werden.

Um Einflüsse des Rauschens bei der Ermittlung der für die Kapazität charakteristischen Größe zu minimieren, wird vorteilhafterweise während deren Ermittlung ein zeitlicher Mittelwert gebildet. Hierdurch werden kurzfristige, durch Rauschen verursachte Sprünge ausgeglichen.
Ein weiterer Vorteil der oben beschriebenen absoluten Kapazitätsmessung im Vergleich zum Stand der Technik ist die Möglichkeit, zusätzliche Fehler, welche nicht direkt mit der Detektion von Oberflächen zu tun haben, zu detektieren. Dazu wird vorteilhafterweise bei Unterschreiten eines vorgegebenen ersten Referenzwerts der Größe und/oder bei Überschreiten eines vorgegebenen zweiten Referenzwerts der Größe ein Fehlersignal ausgegeben. Ist nämlich der kapazitive Ruhewert, verglichen mit einem bekannten Referenzwert, zu klein, so ist die Pipettiernadel nicht vorhanden oder nicht korrekt angeschlossen. Ist der kapazitive Ruhewert, verglichen mit einem bekannten Referenzwert, zu groß, so ist die Pipettiernadel defekt oder berührt eine leitfähige Oberfläche.
Als für die Kapazität charakteristische Größe können verschiedene, im Wesentlichen mit der während des Auf- und Entladens übertragenen Ladung verknüpfte Messgrößen verwendet werden. Die erfindungsgemässe Ausgestaltung des Verfahrens ergibt sich jedoch, indem als Größe die Anzahl der Auf- und Entladevorgänge innerhalb eines vorbestimmten Zeitraums verwendet wird. Je größer die Kapazität, desto länger dauert der Aufladevorgang bis zu einer vorgegebenen Spannung.
Eine Ermittlung dieser Größe wird besonders einfach vorteilhafterweise dadurch erreicht, dass das Aufladen mittels einer konstanten Stromquelle erfolgt, das Entladen bei Erreichen einer vorgegebenen Spannung gestartet wird, und ein neuer Zyklus nach Erreichen einer Nullspannung mit erneutem Aufladen gestartet wird. Hierzu kann ein Komparator innerhalb der hierfür verantwortlichen elektrischen Schaltung vorgesehen sein.
Eine Berührungserkennungseinrichtung für ein In-Vitro-Diagnostiksystem wird in dem beschriebenen Verfahren verwendet. Ein in dem Verfahren verwendetes In-Vitro-Diagnostiksystem umfasst eine derartige Berührungserkennungseinrichtung.

Die mit der Erfindung erzielten Vorteile bestehen insbesondere darin, dass durch eine Berührungserkennung auf Basis der absoluten Kapazität zwischen Pipettiernadel und einem Referenzpotenzial Fehlsignale durch elektromagnetische Störungen weitestgehend eliminiert werden und dadurch ein zuverlässigeres Pipettieren in einem In-Vitro-Diagnostiksystem möglich ist. Dieser Ansatz erspart zudem eine spezielle Messelektrode, da die für den Flüssigkeitstransport eingesetzte Pipettiernadel direkt verwendet werden kann. Weiterhin können zuverlässig Fehlerzustände wie eine nicht angeschlossene Nadel oder eine Berührung einer leitenden Oberfläche erkannt werden. Dies kann z. B. auch zur Justage von beweglichen Teilen (z. B. Transportarmen) verwendet werden.

Ein Ausführungsbeispiel der Erfindung wird anhand einer Zeichnung näher erläutert. Darin zeigen:
- FIG 1: eine schematische Darstellung eines In-Vitro-Diagnostiksystems,
- FIG 2: eine schematische Darstellung einer an einem Transportarm befestigten Pipettiernadel im In-Vitro-Diagnostiksystem,
- FIG 3: einen Graphen des Verlaufs der für die Kapazität charakteristischen Größe beim Eintauch- und Auftauchvorgang der Pipettiernadel,
- FIG 4: eine ausschnittsweise Vergrößerung des Graphen aus FIG 3,
- FIG 5: einen Graphen des Verlaufs der für die Kapazität charakteristischen Größe im Falle eines elektromagnetischen Störsignals, und
- FIG 6: einen Graphen des Verlaufs der für die Kapazität charakteristischen Größe im Falle einer Störung der Kontinuität, z. B. durch einen Wackelkontakt.

Gleiche Teile sind in allen Figuren mit denselben Bezugszeichen versehen.

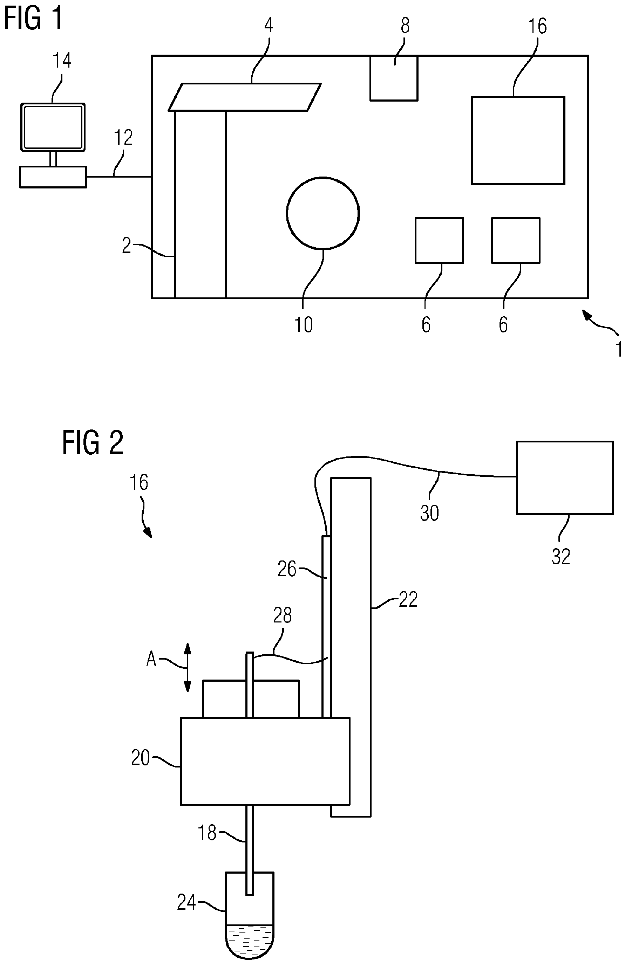

FIG 1 zeigt eine schematische Darstellung eines In-Vitro-Diagnostiksystems 1 mit einigen darin enthaltenen Bauteilen. Hierbei werden nur die wichtigsten Bauteile stark vereinfacht dargestellt, um die grundsätzliche Funktion des In-Vitro-Diagnostiksystems 1 zu erläutern, ohne hierbei detailliert die einzelnen Teile jedes Bauteils darzustellen.

Das In-Vitro-Diagnostiksystem 1 ist dafür ausgebildet, vollautomatisch verschiedenste Analysen von Blut oder anderen Körperflüssigkeiten durchzuführen, ohne dass hierfür Aktivitäten eines Benutzers notwendig wären. Diese beschränken sich vielmehr auf Wartung oder Reparatur und Nachfüllarbeiten, wenn z. B. Küvetten oder Reagenzien nachgefüllt werden müssen.

Die Proben werden dem In-Vitro-Diagnostiksystem 1 auf nicht näher dargestellten Schlitten in einer Zuführungsschiene 2 zugeführt. Informationen hinsichtlich der pro Probe durchzuführenden Analysen können hierbei beispielsweise mittels auf den Probengefäßen angebrachten Strichcodes übergeben werden, die im In-Vitro-Diagnostiksystem 1 ausgelesen werden. Aus den Proben werden in einer Pipettiereinrichtung 4 Aliquots mittels einer noch in FIG 2 näher dargestellten Pipettiernadel entnommen.

Die Aliquots werden ebenfalls nicht näher dargestellten Küvetten zugeführt, in denen die eigentlichen Analysen mittels verschiedenster Messgeräte 6 wie z. B. Photometern etc. durchgeführt werden. Die Küvetten werden aus einem Küvettenspeicher 8 entnommen. Zusätzlich können aus einem Reagenzienspeicher 10 mittels einer weiteren, wie bereits erwähnt in FIG 2 dargestellten Pipettiernadel weitere Reagenzien der jeweiligen Küvette zugeführt werden, die je nach durchzuführender Analyse erforderlich sind.

Der Transport der Küvetten innerhalb des In-Vitro-Diagnostiksystems erfolgt mit hier nicht näher dargestellten Transporteinrichtungen wie z. B. Transferarmen, die in verschiedenste Raumrichtungen bewegbar sind und eine Greifeinrichtung zum Erfassen der Küvetten aufweisen. Der gesamte Prozess wird von einer Steuerungseinrichtung wie z. B. einem über eine Datenleitung 12 angeschlossenen Rechner 14 gesteuert, unterstützt durch eine Vielzahl weiterer, nicht näher dargestellter elektronischer Schaltungen und Mikroprozessoren innerhalb des In-Vitro-Diagnostiksystems 1 und seiner Bauteile.

FIG 2 zeigt schematisch eine der im In-Vitro-Diagnostiksystem 1 vorhandenen Pipettiervorrichtungen 16 mit einer Pipettiernadel 18. Die Pipettiernadel 18 ist über eine Halterung 20 an einem Transferarm 22 befestigt und ist zumindest in Bewegungsrichtung A entlang der Achse der Pipettiernadel 18 automatisiert bewegbar, um in ein Gefäß 24 mit einer Flüssigkeit eingeführt werden zu können und die Flüssigkeit zu pipettieren.

Zur Erkennung der Berührung der Flüssigkeitsoberfläche beim Pipettieren weist das In-Vitro-Diagnostiksystem 1 eine Berührungserkennungseinrichtung auf, welche zunächst eine Leiterplatte 26 mit einer elektrischen Schaltung aufweist, die im Ergebnis absolute Kapazitätsmessungen in oberen Femto- oder unteren Picofarad Bereich erlaubt und im Folgenden beschrieben wird:
Die elektrische Schaltung der Leiterplatte 26 umfasst eine Konstantstromquelle, welche über eine elektrische Verbindung 28 eine Ladung auf die Pipettiernadel 18 aufbringen kann. Die Pipettiernadel 18 bildet gegenüber dem Erd-Nullniveau, das als Referenzpotenzial herangezogen wird, eine zu messende Kapazität, die somit geladen wird. Weiterhin weist die elektrische Schaltung einen Widerstand auf, mit dem die Kapazität wieder entladen werden kann, d. h. über die elektrische Verbindung 28 die Ladung von der Pipettiernadel 18 wieder abgeführt werden kann. Weiterhin weist die elektrische Schaltung einen Komparator auf, welcher den Ladezustand signalisiert und damit insbesondere das Erreichen einer bestimmten Spannung zwischen Pipettiernadel 18 und Erd-Nullniveau anzeigen kann.

Eine digitale Steuerung führt nun kontinuierlich viele zyklische Lade- und Entladevorgänge durch, die jeweils nur 1-10 µs dauern. Die Pipettiernadel 18 wird dabei aufgeladen, bis ein bestimmter Spannungswert erreicht ist, und bei Erreichen des Spannungswerts wieder entladen, bis eine Nullspannung erreicht ist. Dann beginnt ein neuer Ladevorgang. In einer Ausführungsform gibt die Leiterplatte 26 für jeden Ladevorgang ein Signal über die Verbindung 30 an eine Steuerungseinrichtung 32 weiter. In der im Folgenden weiter beschriebenen Ausführungsform wertet die Leiterplatte 26 als integrierter Schaltkreis jedoch die gesamte Kapazitätsmessung mit Zählung usw. aus und gibt nur das Ergebnis, d.h. die Kapazitätsgröße an die Steuereinrichtung 32 weiter. Bei der Steuerungseinrichtung 32 kann es sich um den Rechner 14 aus FIG 1 handeln, oder aber um eine vorgeschaltete spezielle Schaltung als Steuerungseinrichtung 32 in Form eines integrierten Schaltkreises handeln, die in das In-Vitro-Diagnostiksystem 1 integriert ist.

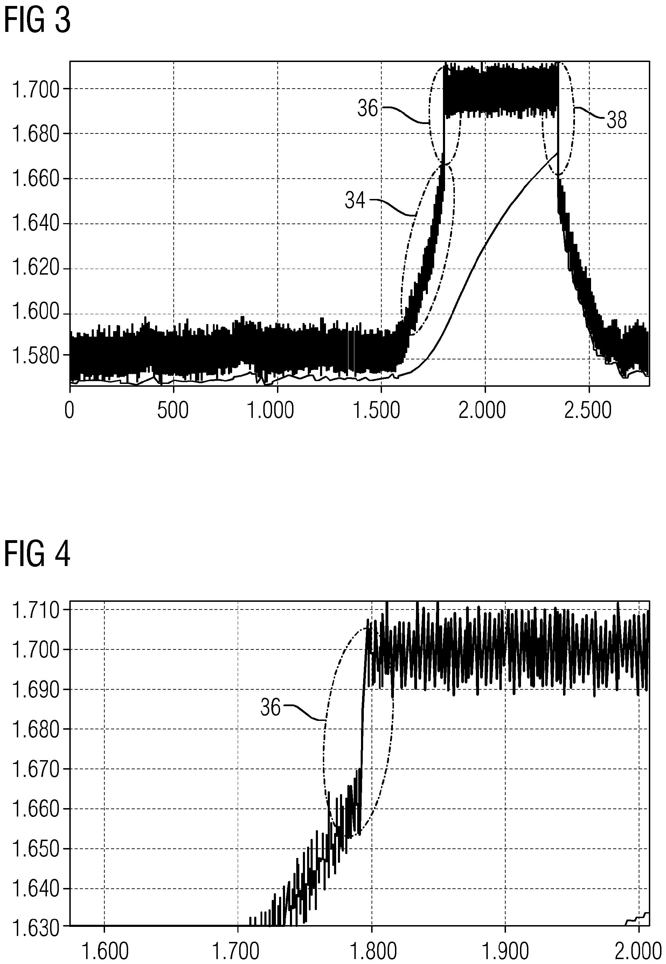

Die Schaltung auf der Leiterplatte 26 steuert und wertet die Lade- und Entladevorgänge aus. Je größer die Kapazität zwischen Pipettiernadel 18 und Erd-Nullniveau ist, desto länger dauert der Ladevorgang. Somit ist die Anzahl der pro Zeiteinheit stattfindenden Lade- und Entladevorgänge eine für die absolute Kapazität charakteristische Größe. Die Schaltung auf Leiterplatte 26 bestimmt über eine Mittelwertbildung, welche beispielsweise in einem Prozessorteil durchgeführt wird, Messwerte der Größe, welche die Kapazität repräsentiert. Bei diesen Messwerten handelt es sich um einheitenlose Zahlenwerte, die die gemessene Kapazität charakterisieren. Diese Zahlenwerte sind in den FIG 3 bis 6 aufgetragen gegen die Zeit in ms. Für die Anwendung im Bereich der Berührungsdetektion des Flüssigkeitsstands ist eine Kalibrierung der Schaltung auf eine absolute Größe in Farad nicht notwendig.

In der Steuerungseinrichtung 32 wird die Berührung mittels eines Auswertealgorithmus der über die Verbindung 30 eingehenden Signale erkannt. Der Auswertealgorithmus analysiert hierbei den Verlauf der für den Kapazitätswert der Pipettiernadel 18 charakteristischen Größe und analysiert ihn nach verschiedenen Aspekten, wie Absolutwert, Geschwindigkeit von Änderungen, Steigung der Änderungen und Kontinuität der Änderungen. Verläufe dieser Größe sind in den FIG 3 bis FIG 6 gegen die Zeit als Graph aufgetragen.

Grundprinzip des Algorithmus ist die Tatsache, dass die gemessene Kapazität beim Eintauchen der Nadel oder bei Kontakt mit einer leitfähigen Oberfläche größer wird. FIG 3 zeigt, wie sich das Kapazitätssignal beim Eintauchen in ein Probenröhrchen (gleichmäßiger Anstieg im Bereich 34) und der anschließenden Berührung der Flüssigkeitsoberfläche (sprunghafte Änderung im Bereich 36) verhält. Der Bereich 36 ist in FIG 4 vergrößert dargestellt. Gut erkennbar ist auch das überlagerte Signalrauschen, welches die Auswertbarkeit der Nutzsignale aber nicht beeinträchtigt.

Im Moment der Berührung im Bereich 36 ändert sich die Kapazität sprunghaft und verharrt dann auf dem neuen Wert. Dieser Vorgang gilt natürlich auch umgekehrt, wenn die Flüssigkeit verlassen wird, oder die Berührung endet (siehe Bereich 38 in FIG 3). Der Algorithmus wertet dafür den Verlauf der Größe aus. Er sucht dabei nach Sprungstellen, d. h. nach Bereichen, in denen die Größe innerhalb einer kurzen Zeit von weniger als 1 ms über einen vorbestimmten Mindestwert ansteigt, und überprüft weiter, ob und wie lange das neue Signalniveau bestehen bleibt. Hierzu ist in einer Ausführungsform vorgesehen, dass der Verlauf innerhalb von mehr als 10 ms innerhalb eines bestimmten Bereichs bleiben muss. Bei der Festlegung von Mindestwerten und Bereich kann die Stärke des Signalrauschens dynamisch einbezogen werden. Alternativ können feste Werte vorgegeben werden.

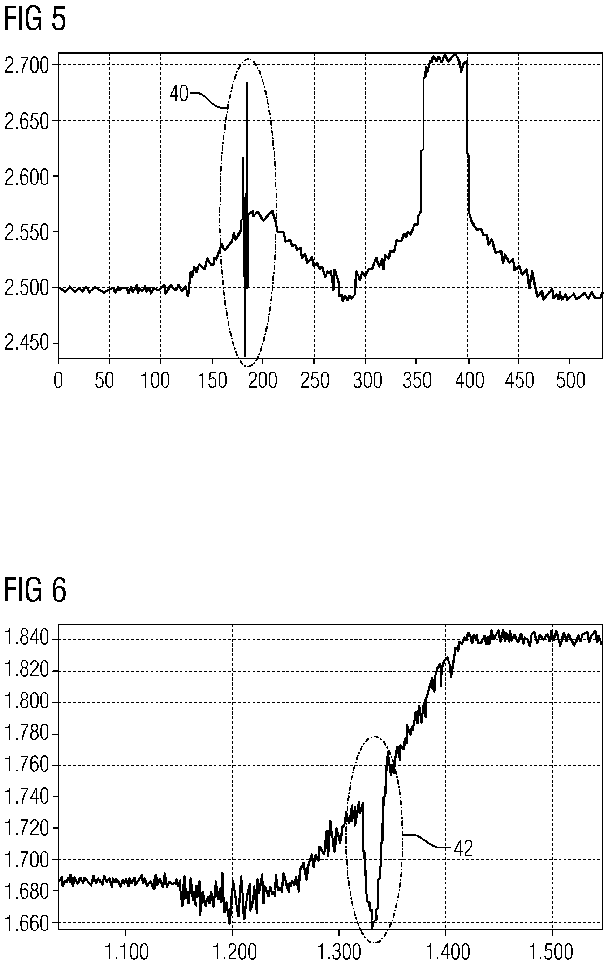

Mit Hilfe des Algorithmus kann eine Störung von einer korrekten Berührung der Oberfläche der Flüssigkeit unterschieden werden. Die FIG 5 und 6 zeigen zwei typische Störszenarien. FIG 5 zeigt im Bereich 40 eine punktuelle Überhöhung der Rauschamplitude durch eine elektromagnetische Störung. FIG 6 zeigt im Bereich 42 einen kurzen Abfall des Verlaufs der Größe, z. B. durch einen Wackelkontakt. Durch die kurze Dauer werden diese Signale jedoch nicht als Berührungssignale missinterpretiert. Es ist gut zu erkennen, das auch hier die Nutzsignale auswertbar bleiben. Beide Fälle hätten mit dem Stand der Technik (reine Detektion von Kapazitätsänderungen) zu Fehlmessungen geführt.

Nicht genauer abgebildet ist eine weitere Funktion des Algorithmus: Es werden Referenzwerte vorgegeben, bei deren jeweiligem überschreiten oder unterschreiten jeweils ein Fehlersignal ausgegeben wird: Ist nämlich der kapazitive Ruhewert, verglichen mit einem bekannten Referenzwert, zu klein, so ist der die Pipettiernadel 18 nicht vorhanden oder nicht korrekt angeschlossen. Ist der kapazitive Ruhewert, verglichen mit einem bekannten Referenzwert, zu groß, so ist die Pipettiernadel 18 defekt oder berührt eine leitfähige Oberfläche. Letztere Signalisierung kann auch zur Justage der Pipettiernadel 18 verwendet werden.

### Bezugszeichenliste

- 1: In-Vitro-Diagnostiksystem
- 2: Zuführungsschiene
- 4: Pipettiereinrichtung
- 6: Messgerät
- 8: Küvettenspeicher
- 10: Reagenzienspeicher
- 12: Datenleitung
- 14: Rechner
- 16: Pipettiervorrichtung
- 18: Pipettiernadel
- 20: Halterung
- 22: Transferarm
- 24: Gefäß
- 26: Leiterplatte
- 28: elektrische Verbindung
- 30: Verbindung
- 32: Steuerungseinrichtung
- 34, 36:
- 38, 40:
- 42: Bereiche kapazitiver Signalverläufe

- A: Bewegungsrichtung

## Patentansprüche

1. Verfahren zur Berührungserkennung einer Pipettiernadel in einem In-Vitro-Diagnostiksystem (1), bei dem
- die Pipettiernadel (18) zyklisch durch eine zwischen Pipettiernadel (18) und einem Referenzpotenzial angelegte elektrische Spannung oder Strom aufgeladen und durch anschließende elektrische Verbindung der Pipettiernadel (18) mit dem Referenzpotenzial wieder entladen wird,
- aus einer Anzahl von während des Aufladens und/oder Entladens erfasster Messwerte eine für die aktuelle Kapazität zwischen Pipettiernadel (18) und Referenzpotenzial charakteristische Größe ermittelt wird, und
- der zeitliche Verlauf der Größe anhand einer Anzahl vorgegebener Kriterien kontinuierlich überprüft wird und bei Erfüllung der vorgegebenen Kriterien ein Berührungssignal erzeugt wird,
**dadurch gekennzeichnet, dass** als die für die Kapazität zwischen Pipettiernadel (18) und Referenzpotenzial charakteristische Größe die Anzahl der Auf- und Entladevorgänge innerhalb eines vorbestimmten Zeitraums verwendet wird.

2. Verfahren nach Anspruch 1, bei dem ein vorgegebenes Kriterium dadurch gegeben ist, dass innerhalb eines vorgegebenen Zeitraumes eine Erhöhung der Größe um einen Mindestwert erfolgt.

3. Verfahren nach Anspruch 2, bei dem der vorgegebene Zeitraum weniger als 10 ms, insbesondere weniger als 5 ms beträgt.

4. Verfahren nach Anspruch 2 oder 3, bei dem ein fester Mindestwert vorgegeben wird.

5. Verfahren nach Anspruch 2 oder 3, bei dem der Mindestwert in Abhängigkeit von der Stärke eines Rauschens der Größe bestimmt wird.

6. Verfahren nach einem der Ansprüche 2 bis 5, bei dem ein zusätzliches Kriterium dadurch gegeben ist, dass die Größe im zeitlichen Anschluss an den vorgegebenen Zeitraum für einen zweiten vorgegebenen Zeitraum innerhalb eines Bereichs konstant bleibt.

7. Verfahren nach Anspruch 6, bei dem der zweite vorgegebene Zeitraum mehr als 10 ms beträgt.

8. Verfahren nach Anspruch 6 oder 7, bei dem ein fester Bereich vorgegeben wird.

9. Verfahren nach Anspruch 6 oder 7, bei dem der Bereich in Abhängigkeit von der Stärke eines Rauschens der Größe bestimmt wird.

10. Verfahren nach einem der vorhergehenden Ansprüche, bei dem bei der Ermittlung der Größe ein zeitlicher Mittelwert gebildet wird.

11. Verfahren nach einem der vorhergehenden Ansprüche, bei dem bei Unterschreiten eines vorgegebenen ersten Referenzwerts der Größe und/oder bei Überschreiten eines vorgegebenen zweiten Referenzwerts der Größe ein Fehlersignal ausgegeben wird.

12. Verfahren nach einem der vorhergehenden Ansprüche, bei dem das Aufladen mittels einer konstanten Stromquelle erfolgt, das Entladen bei Erreichen einer vorgegebenen Spannung gestartet wird, und ein neuer Zyklus nach Erreichen einer Nullspannung mit erneutem Aufladen gestartet wird.

13. Verwendung einer Berührungserkennungseinrichtung für ein In-Vitro-Diagnostiksystem (1) umfassend eine Konstantstromquelle, eine Pipettiernadel, einen Widerstand und einen Komparator in dem Verfahren nach einem der vorhergehenden Ansprüche.

14. Verwendung eines In-Vitro-Diagnostiksystems (1) mit einer Berührungserkennungseinrichtung in dem Verfahren nach einem der Ansprüche 1 bis 12, die Berührungserkennungseinrichtung umfassend eine Konstantstromquelle, eine Pipettiernadel, einen Widerstand und einen Komparator.

## Claims

1. Method for detecting contact of a pipetting needle in an in vitro diagnostic system (1), wherein
- the pipetting needle (18) is cyclically charged by an electric voltage or current applied between pipetting needle (18) and a reference potential and discharged again by a subsequent electric connection between the pipetting needle (18) and the reference potential,
- a characteristic variable for the current capacitance between pipetting needle (18) and reference potential is established from a number of measured values detected during the charging and/or discharging, and
- the temporal curve of the variable is monitored continuously on the basis of a number of predetermined criteria and a contact signal is generated if the predetermined criteria are satisfied,
**characterized in that** the number of charging and discharging processes within a predetermined period of time is used as the characteristic variable for the capacitance between pipetting needle (18) and reference potential.

2. Method according to Claim 1, wherein a predetermined criterion is given by virtue of the variable being increased by a minimum value within a predetermined period of time.

3. Method according to Claim 2, wherein the predetermined period of time is less than 10 ms, in particular less than 5 ms.

4. Method according to Claim 2 or 3, wherein a fixed minimum value is predetermined.

5. Method according to Claim 2 or 3, wherein the minimum value is determined in a manner dependent on the strength of a noise of the variable.

6. Method according to one of Claims 2 to 5, wherein an additional criterion is given by virtue of the variable remaining constant within a range for a second predetermined period of time following the predetermined period of time in time.

7. Method according to Claim 6, wherein the second predetermined period of time is more than 10 ms.

8. Method according to Claim 6 or 7, wherein a fixed range is predetermined.

9. Method according to Claim 6 or 7, wherein the range is determined in a manner dependent on the strength of a noise of the variable.

10. Method according to one of the preceding claims, wherein a time average value is formed when establishing the variable.

11. Method according to one of the preceding claims, wherein an error signal is output if a predetermined first reference value of the variable is undershot and/or if a predetermined second reference value of the variable is exceeded.

12. Method according to one of the preceding claims, wherein charging is carried out by means of a constant current source, discharging is started once a predetermined voltage is reached and a new cycle is started with renewed charging after reaching a zero voltage.

13. Use of a contact detection apparatus for an in vitro diagnostic system (1), comprising a constant current source, a pipetting needle, a resistor and a comparator, in the method according to one of the preceding claims.

14. Use of an in vitro diagnostic system (1) comprising a contact detection apparatus in the method according to one of Claims 1 to 12, the contact detection apparatus comprising a constant current source, a pipetting needle, a resistor and a comparator.

## Revendications

1. Procédé de détection de contact d'une aiguille de pipetage dans un système (1) de diagnostic in vitro, dans lequel
- on charge l'aiguille (18) de pipetage cycliquement par une tension ou un courant électrique appliqué entre l'aiguille (18) de pipetage et un potentiel de référence et on la redécharge en reliant ensuite électriquement l'aiguille (18) de pipetage au potentiel de référence,
- à partir d'un certain nombre de valeurs de mesure détectées pendant la charge et/ou la décharge, on détermine une grandeur caractéristique de la capacité instantanée entre l'aiguille (18) de pipetage et le potentiel de référence et
- on contrôle continuellement la variation dans le temps de la grandeur à l'aide d'un certain nombre de critères donnés à l'avance et, lorsque les critères donnés à l'avance sont satisfaits, on produit un signal de contact,
**caractérisé en ce que** l'on utilise, comme grandeur caractéristique de la capacité entre l'aiguille (18) de pipetage et le potentiel de référence, le nombre des opérations de charge et de décharge dans un laps de temps défini à l'avance.

2. Procédé suivant la revendication 1, dans lequel un critère donné à l'avance est donné par le fait qu'il se produit, dans un laps de temps donné à l'avance, une augmentation d'une valeur minimum de la grandeur.

3. Procédé suivant la revendication 2, dans lequel le laps de temps donné à l'avance est plus petit que 10 ms, notamment plus petit que 5 ms.

4. Procédé suivant la revendication 2 ou 3, dans lequel on donne à l'avance une valeur minimum fixe.

5. Procédé suivant la revendication 2 ou 3, dans lequel on définit la valeur minimum en fonction de l'intensité d'un bruit de la grandeur.

6. Procédé suivant l'une des revendications 2 à 5, dans lequel un critère supplémentaire est donné par le fait que la grandeur reste, dans la suite dans le temps du laps de temps donné à l'avance, constante dans une plage pendant un deuxième laps de temps donné à l'avance.

7. Procédé suivant la revendication 6, dans lequel le deuxième laps de temps donné à l'avance est supérieur à 10 ms.

8. Procédé suivant la revendication 6 ou 7, dans lequel on donne à l'avance une plage fixe.

9. Procédé suivant la revendication 6 ou 7, dans lequel on définit la plage en fonction de l'intensité d'un bruit de la grandeur.

10. Procédé suivant l'une des revendications précédentes, dans lequel, dans la détermination de la grandeur, on forme une valeur moyenne en fonction du temps.

11. Procédé suivant l'une des revendications précédentes, dans lequel, si l'on n'atteint pas une première valeur de référence donnée à l'avance de la grandeur et/ou si une deuxième valeur de référence donnée à l'avance de la grandeur est dépassée, on émet un signal d'erreur.

12. Procédé suivant l'une des revendications précédentes, dans lequel la charge s'effectue au moyen d'une source de courant constante, on lance la décharge lorsqu'une tension donnée à l'avance est atteinte et on lance un nouveau cycle après que la tension zéro est atteinte.

13. Utilisation d'un dispositif de détection de contact pour un système (1) de diagnostic in vitro, comprenant une source de courant constante, une aiguille de pipetage, une résistance et un comparateur dans le procédé suivant l'une des revendications précédentes.

14. Utilisation d'un système (1) de diagnostic in vitro, comprenant un dispositif de détection de contact dans le procédé suivant l'une des revendications 1 à 12, le dispositif de détection de contact comprenant une source de courant constante, une aiguille de pipetage, une résistance et un comparateur.
